# EUROPEAN PATENT APPLICATION

(11) **EP 2 345 408 A2**
(43) Date of publication of application: **20.07.2011**
(21) Application number: 11250010.3
(22) Date of filing: 07.01.2011
(51) Int. Cl.: A61K 9/28, A61K 9/48, A61K 9/50, A61K 31/4439, A61K 9/20

(54) **Acid labile drug formulations**

(30) Priority: 08.01.2010 IN CH00642010; 27.05.2010 US 349054 P
(71) Applicant: Dr. Reddy's Laboratories Ltd., Hyderabad 500 016, Andhra Pradesh (IN)
(72) Inventor: Kumar, Ghosh Pradip, Midnapur (West) 721 156 West Bengal (IN); Singh, Rai Amrinder, Patiala 147002 Punjab (IN); Bijayananda, Sahoo, Nayabazar, Bhadrak 756 10 Orissa (IN); Vashdev, Nihalani Girish, Ulhasnagar 421 005 Mumbai, Maharashtra (IN); Premchand, Agrawal Pratit, Gondia 441 701 Maharashtra (IN); Suryakumar, Jayanthi, Madeenaguda,Hyderabad 500 049, Andhra Pradesh (IN); Venkateswarlu, Vobalaboina, Hyderabad 500 072 Andhra Pradesh (IN)
(74) Representative: Bates, Philip Ian

(57) **Abstract**

Pharmaceutical formulations comprise inert cores that are coated with a composition comprising an acid labile drug, further sequentially coated with a barrier or intermediate layer and an enteric coating layer. Coated cores may be further formulated to produce tablets or capsules.

## Description

### INTRODUCTION

Aspects of the present invention relate to pharmaceutical formulations comprising at least one acid labile pharmaceutically active substance, or any salts, polymorphs, hydrates, esters, isomers, derivatives, or mixtures thereof, for oral use, processes for the manufacture of such formulations and methods of preventing or treating diseases or disorders using such formulations. An aspect of the invention provides methods for producing a gastric acid secretion inhibitory effect to a subject in need thereof, by administering an effective amount of the pharmaceutical formulations. Particular embodiments of the present invention comprise at least one substituted benzimidazole derivative, such as esomeprazole, as the active agent. In embodiments, the formulations of the present invention are highly stable, and in order to enhance the storage stability, at least one basic compound such as metal compound and/or at least one alkaline compound is included therein.

Substituted benzimidazoles are potent inhibitors of gastric acid secretion. These compounds are susceptible to degradation and/or transformations in both acid and neutral media. The acidic decomposition of these acid labile compounds is due to an acid catalyzed reaction. Therefore, such labile drugs need to be formulated in a way to stabilize the compositions. The acid-labile drugs comprise substituted benzimidazole gastric anti-secretary agents, such as esomeprazole, omeprazole, and pharmaceutically acceptable salts thereof. These agents are known as proton pump inhibitors with powerful inhibitory action against secretion of gastric acid. They are indicated for the treatment of various digestive ulcers, are well known in the art. Esomeprazole is useful for inhibiting gastric acid secretion and has gastric mucosa protective activity. In a more general sense, esomeprazole may be used for prevention and treatment of gastric acid related disorders in mammals and man, including, e.g. gastroesofageal reflux disease, gastritis, gastric ulcer, duodenal ulcer, etc. Esomeprazole is susceptible to degradation/transformations in acid reacting and neutral media. The stability of esomeprazole is also affected by moisture, heat, and organic solvents, and to some degree by light.

When these compounds are formulated into pharmaceutical preparations for oral administration, they require special techniques to avoid contact of drug with gastric acid of the stomach. One technique most commonly used is to coat the acid-labile compound, such as in its granules, pellets, or tablets, with an enteric polymer coating, which is insoluble in water under acidic conditions and soluble in water under neutral to alkaline conditions. However, the material used in enteric coatings itself is acidic, which can cause the decomposition of the acid-labile compound. Such decomposition occurs even during the enteric coating process, which results in the coloration of the surface of the drug-containing core. In order to avoid such problems, an inert barrier coating, which is not acidic, is often required between the core and enteric coating, which increases the complexity and the cost of the formulation manufacturing processes involving acid-labile compounds.

An acid labile compound can be a substituted benzimidazole derivative having proton pump inhibitor (PPI) activity, such as esomeprazole. Esomeprazole has a chemical name 5-methoxy-2-[(S)-[(4-methoxy-3,5-dimethyl-2-pyridinyl)methyl]sulfinyl]-1*H*-benzimidazole-1yl and has structural Formula I.

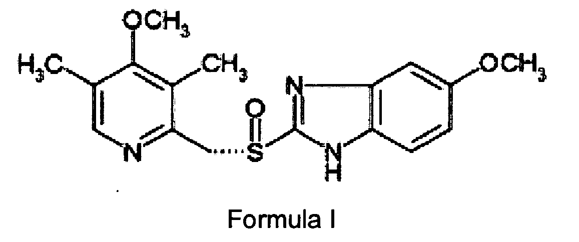

U.S. Patent No. 4,255,431 discloses omeprazole and therapeutically acceptable salts thereof. The advantages of using salts of omeprazole and particularly the magnesium salt are disclosed in U.S. Patent No. 4,738,974. The single isomers of omeprazole are reported to be more useful in therapy when compared to the racemic omeprazole.

European Patent No. 1020461 B1 describes the use of the (-)-omeprazole magnesium salt for the manufacture of a medicament for the inhibition of gastric acid secretion.

European Patent Publication No. 1960384 A1 describes various crystalline esomeprazole non-salt forms.

U.S. Patent No. 5,877,192 discloses the use of the (-)-enantiomer of omeprazole (esomeprazole), or a pharmaceutically acceptable salt thereof, in the treatment of gastric acid related diseases as a means to decrease inter-individual variation in plasma levels, compared to omeprazole.

U.S. Patent No. 5,753,265 describes an oral pharmaceutical composition in the form of a multiple unit tablet comprising acid-labile compounds with an enteric coating layer.

U.S. Patent No. 6,013,281 discloses that a separating layer is formed *in situ* by direct application of an acidic enteric material onto an alkaline core containing benzimidazoles.

U.S. Patent Application Publication No. 2006/0057195 A1 describes stable preparations for medicinal use containing amorphous benzimidazole compounds, which are produced by blending an amorphous benzimidazole compound with a nontoxic base such as a basic inorganic salt.

European Patent No. 0706378 B1 discloses an oral enteric coated formulation containing a core material comprising a magnesium salt of omeprazole.

U.S. Patent No. 5,900,424 discloses an omeprazole magnesium salt having a degree of crystallinity that is higher than 70%, as determined by x-ray powder diffraction.

It is technically very difficult to formulate compositions comprising esomeprazole, due to associated chemical and physical or polymorphic instability. Currently marketed formulations contain esomeprazole in the form of its magnesium salt which are comparatively stable; however, the potential toxicity associated with the presence of alkaline earth metals such as magnesium is undesirable. Hence there remains a need for developing stable pharmaceutical compositions comprising esomeprazole or a pharmaceutically acceptable less toxic salt thereof such as sodium or potassium salts, formed *in situ,* which exhibit appreciable chemical stability during storage.

### SUMMARY

Aspects of the present invention relate to pharmaceutical formulations comprising at least one acid labile pharmaceutically active substance, or any salts, polymorphs, hydrates, esters, isomers, derivatives, or mixtures thereof, for oral administration, and processes for their preparation.

In embodiments, the active agent is contained in formulations as esomeprazole or salt form during manufacture or storage, in an amorphous form, or crystalline form, or mixtures thereof.

In embodiments, the present invention provides stable formulations comprising esomeprazole, together with one or more pharmaceutically acceptable excipients.

In embodiments, multiple unit esomeprazole formulations of the present invention comprise esomeprazole in an amorphous form or crystalline or mixtures thereof as the active agent, together with at least one metal compound and/or at least one alkaline compound, together with one or more other excipients.

In embodiments, multiple unit formulations of esomeprazole are provided, comprising: (1) a pharmacologically inert core; (2) a drug layer over the core comprising esomeprazole or its salt, at least one metal compound and/or at least one alkaline compound, and one or more pharmaceutically acceptable excipients; (3) a barrier coating layer or intermediate layer over the drug layer, comprising at least one pharmaceutically acceptable excipient; (4) an enteric layer over the barrier coating layer or intermediate layer comprising an enteric coating polymer; and (5) optionally an over coating layer over the enteric coating layer.

In embodiments, multiple unit esomeprazole formulations of the present invention are provided, comprising esomeprazole in an amorphous form as the active agent, together with at least one metal compound and/or at least one alkaline compound, and one or more pharmaceutically acceptable excipients, wherein the esomeprazole is at least partially converted into an esomeprazole salt of the alkaline metal *in situ* during the manufacturing process.

In embodiments, multiple unit esomeprazole formulations are provided comprising: (1) a pharmacologically inert core; (2) a drug layer over the core comprising esomeprazole, at least one metal compound and/or at least one alkaline compound, and one or more pharmaceutically acceptable excipients; (3) a barrier coating layer or intermediate layer over the drug layer, comprising at least one pharmaceutically acceptable excipient; (4) an enteric layer over the barrier coating layer or intermediate layer comprising an enteric coating polymer; and (5) an overcoating layer over the enteric coating layer; wherein the esomeprazole composition in the form of multiple unit particles is made into a single unit dosage form such as a tablet or capsule.

In an aspect, the invention provides processes for preparing esomeprazole formulations, embodiments comprising: (1) seal coating sugar spheres using a binder suspension to obtain inert cores; (2) applying an esomeprazole drug containing layer onto the inert cores to obtain drug coated particles; (3) applying a barrier coating layer onto the drug coated particles; (4) applying an enteric coating layer onto the barrier coated particles; and (5) applying an overcoating layer onto the enteric coated particles.

In an aspect, the invention provides processes for preparing a single unit dosage form of esomeprazole, embodiments comprising: (1) seal coating sugar spheres using a binder suspension to obtain inert cores; (2) applying esomeprazole drug containing layer onto the inert cores to obtain drug coated particles; (3) applying a barrier coating layer onto the drug coated particles; (4) applying an enteric coating layer onto the barrier coated particles; (5) optionally, applying an overcoating layer onto the enteric coated particles to obtain overcoated multiple unit particles; (6) co-granulating the enteric coated or overcoated multiple unit particles with one or more pharmaceutically acceptable excipients; and (7) making the composition thus obtained into a single unit dosage form.

In embodiments, the invention provides processes for producing stable granules or pellets, including drying granules or pellets at temperatures of 50 ±20°C.

In embodiments, esomeprazole compositions of the present invention are in the form of particles. In embodiments, particles according to the present invention may be in the form of powders, granules, pellets, spheroids, extrudates, mini-tablets, and the like.

In embodiments, the invention provides processes for producing stable granules or pellets, wherein granules or pellets have a water content of 0.5-10% by weight, as determined using a Karl Fischer method.

In embodiments, the invention provides processes for producing stable granules or pellets, wherein granules or pellets have loss on drying in the range of about 0.25-10%, or about 0.5-5%, by weight.

In embodiments, the invention includes pharmaceutical formulations containing about 40 mg of esomeprazole and producing esomeprazole Cₘₐₓ values about 50 ng/mL to about 500 ng/mL, AUC(₀₋ₜ) values about 60 ng•hour/mL to about 700 ng•hour/mL, and AUC_{(0-∞)} values about 60 ng•hour/mL to about 900 ng•hour/mL, in plasma after oral administration of a single dose to healthy humans under fasting conditions.

In embodiments, the invention includes pharmaceutical formulations containing about 40 mg of esomeprazole and producing: esomeprazole Cₘₐₓ values about 30 ng/mL to about 300 ng/mL, AUC₍₀₋ₜ₎ values about 40 ng•hour/mL to about 500 ng•hour/mL, and AUC_{(0-∞)} values about 40 ng•hour/mL to about 900 ng•hour/mL, in plasma after oral administration of a single dose to healthy humans under fed conditions.

In embodiments, the present invention provides esomeprazole compositions prepared using esomeprazole or a salt, or mixtures thereof, having particle size distributions with D₅₀ about 0.01 µm to about 100 µm, D₁₀ about 0.001 µm to about 70 µm, and D₉₀ about 0.1 µm to about 700 µm.

In embodiments, the invention provides stable pharmaceutical compositions comprising esomeprazole or pharmaceutically acceptable salts thereof and at least one pharmaceutically acceptable excipient, wherein pH values of the compositions are less than about 14 when dispersed in water.

In an aspect, compositions of the present invention exhibit appreciable chemical storage stability.

In embodiments, the present invention provides storage stable formulations comprising esomeprazole or its pharmaceutically acceptable salts, which are substantially free of degradation impurities.

In embodiments, formulations of the present invention may contain any one or more of impurities 1, 2, 3, 4, 5, A, B, and C, described below, or any other drug-related impurity, in amounts such that any such impurity does not substantially adversely affect the safety of the composition.

In embodiments, the invention provides stable compositions comprising esomeprazole or its pharmaceutically acceptable salts, wherein levels of one or more of impurities 1, 2, 3, 4, and 5, as described herein, are less than about 5%, or less than about 1 %, of the label esomeprazole content.

In embodiments, the invention provides storage stable compositions comprising esomeprazole or its pharmaceutically acceptable salts, wherein levels of one or more of impuritiesy A, B, and C, as described herein, are less than about 5%, or less than about 1%, of the label esomeprazole content.

In embodiments, the invention relates to stable compositions wherein total drug-related impurities, as determined using high performance liquid chromatography (HPLC), are less than about 7%, or less than about 3%, of the label esomeprazole content.

In embodiments, the invention provides processes for producing stable granules or pellets, wherein granules or pellets are processed in an environment where the relative humidity (RH) is not more than about 70%.

An aspect of the present invention provides methods of preventing or treating diseases or disorders using formulations of the present invention comprising at least one substituted benzimidazole derivative or its salts, or mixtures thereof.

An aspect of the invention provides methods for producing a gastric acid secretion inhibitory effect in a subject in need thereof, by administering an effective amount of a pharmaceutical formulations comprising at least one substituted benzimidazole derivative or salts or mixtures thereof.

### BRIEF DESCRIPTION OF THE DRAWINGS

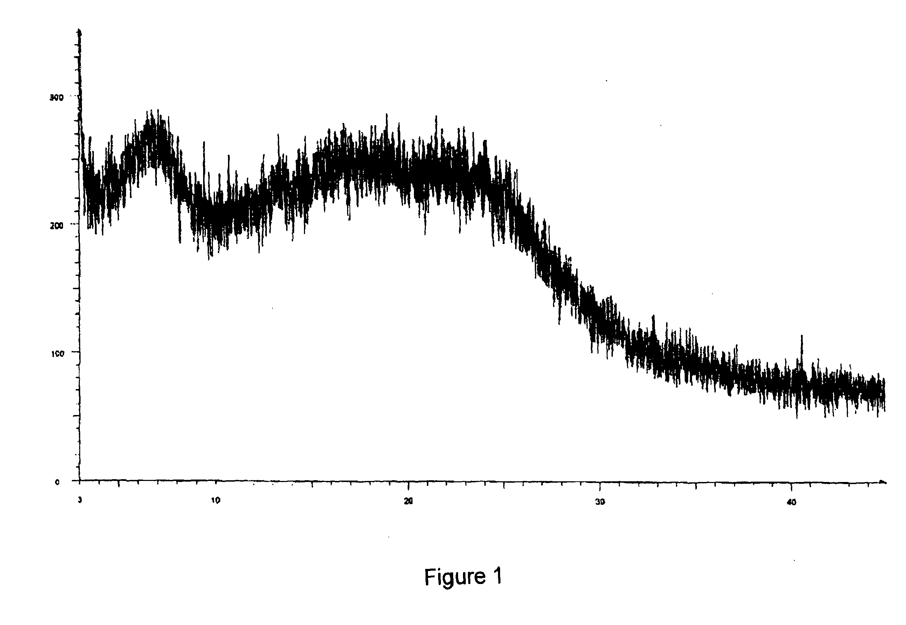

Figure 1 is a powder X-ray diffraction (PXRD) pattern of amorphous esomeprazole that is used to prepare pharmaceutical compositions of the examples.

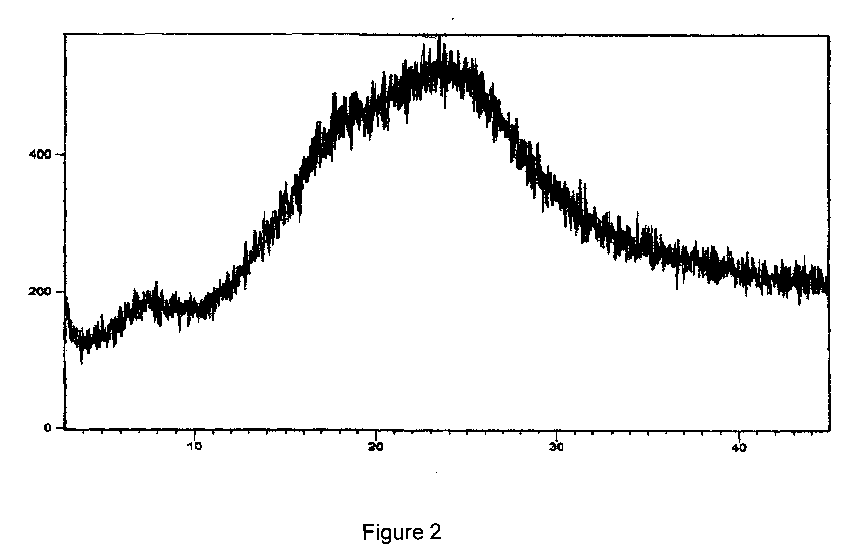

Figure 2 is a PXRD pattern of amorphous esomeprazole after storage for 1 month at 40°C and 75% relative humidity (RH).

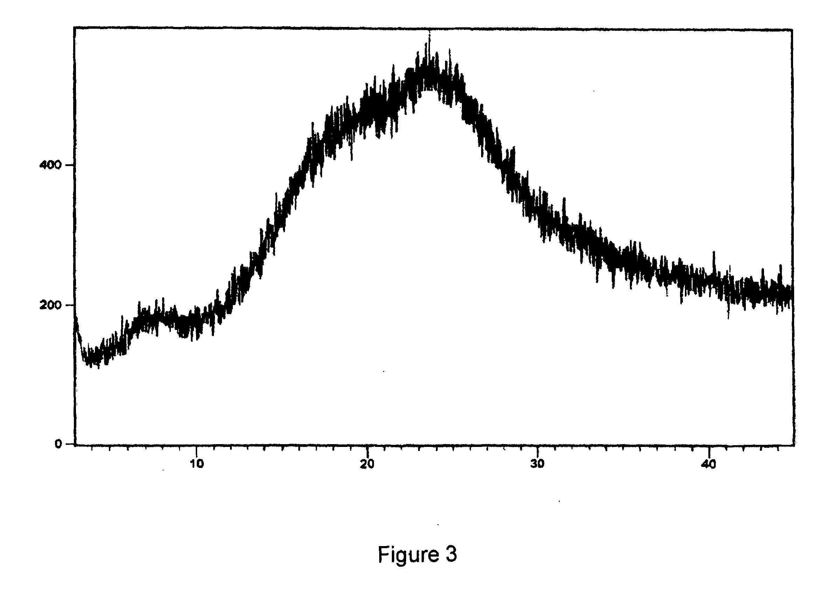

Figure 3 is a PXRD pattern of amorphous esomeprazole after storage for 1 month at 30°C and 65% RH.

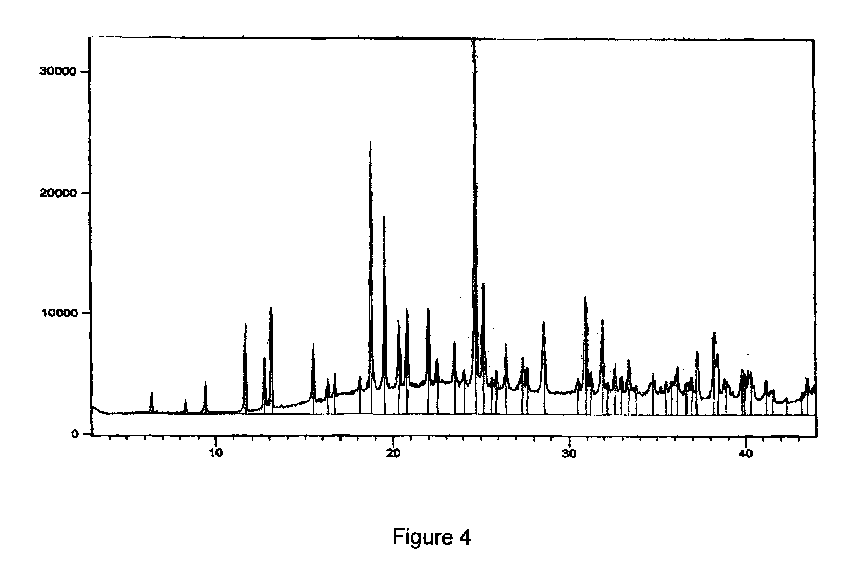

Figure 4 is a PXRD pattern of esomeprazole drug coated pellets prepared in Example 3.

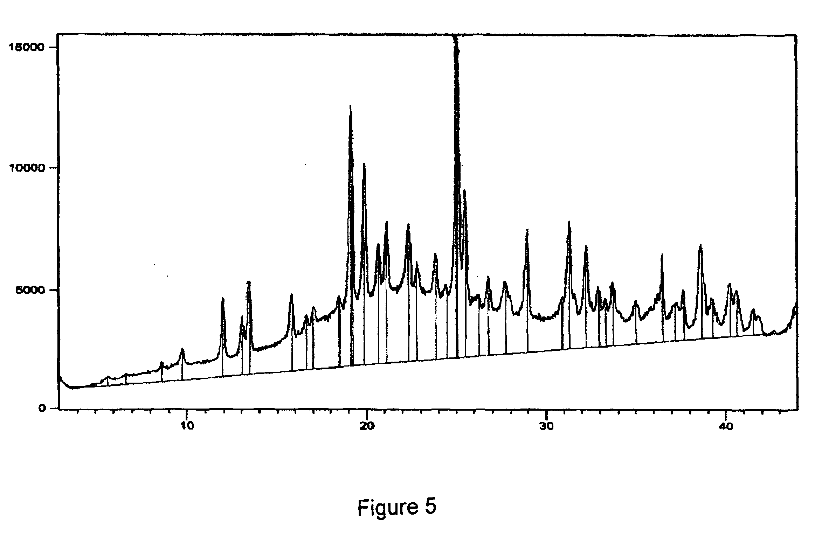

Figure 5 is a PXRD pattern of esomeprazole barrier coated pellets prepared in Example 3.

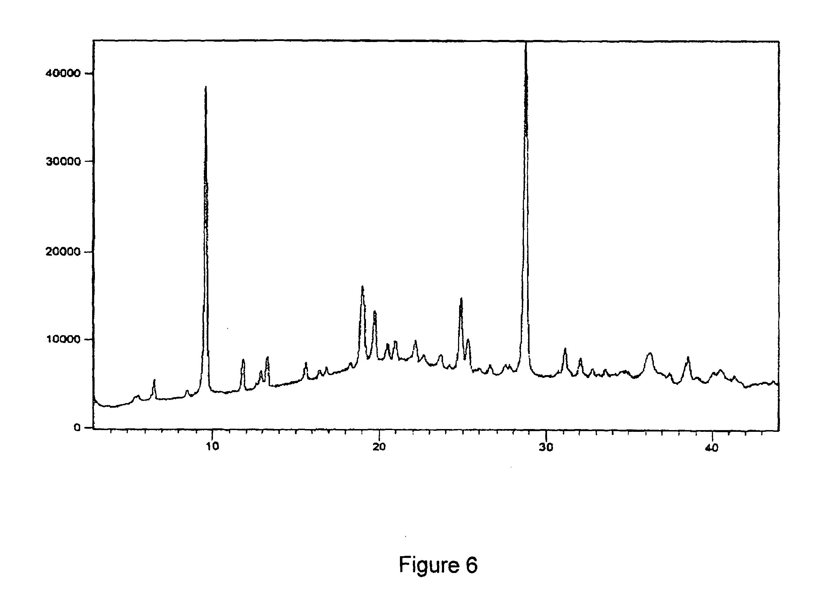

Figure 6 is a PXRD pattern of a pharmaceutical product as prepared in Example 3, containing esomeprazole as the active ingredient.

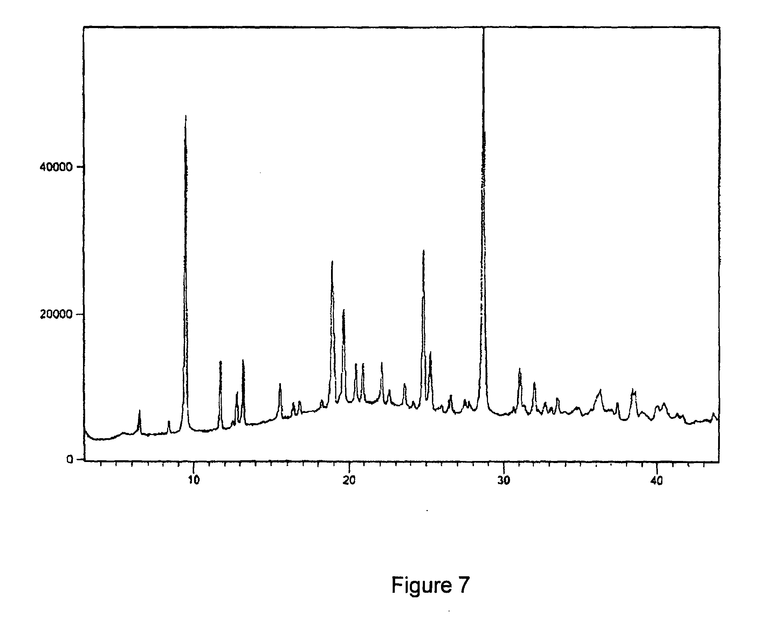

Figure 7 is a PXRD pattern of a pharmaceutical product as prepared in Example 3, after storage for 1 month at 40°C and 75% RH.

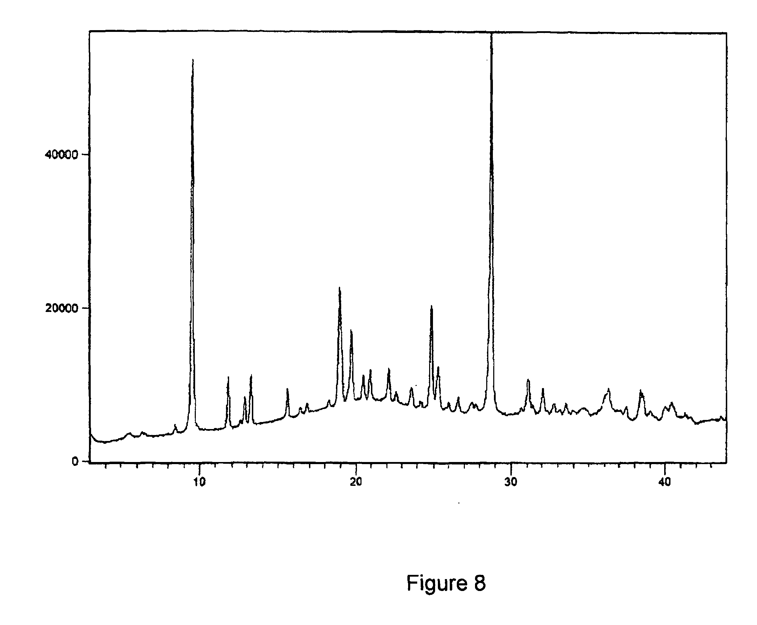

Figure 8 is a PXRD pattern of a pharmaceutical product as prepared in Example 3, after storage for 2 months at 40°C and 75% RH.

All PXRD patterns are generated using copper Kα radiation. The y-axis is in intensity units and the x-axis is the two-theta angle, in degrees.

### DETAILED DESCRIPTION

The term "benzimidazole compound" as used herein refers to any of the compounds belonging to the category of substituted benzimidazoles used for treating gastrointestinal disorders, including esomeprazole, lansoprazole, rabeprazole, pantoprazole, leminoprazole and pariprazole, including their single enantiomers and pharmaceutically acceptable salts, solvates and mixtures. For example, the benzimidazole compound may be esomeprazole, or a pharmaceutically acceptable salt thereof, in any polymorphic form. To simplify the discussion, esomeprazole is used herein to represent this class of drugs, although any of the other drugs also may be used in the invention.

Like other substituted benzimidazole derivatives, esomeprazole is acid-labile, creating several problems in formulating into oral pharmaceutical dosage forms because of the acidic environment of the stomach. It has poor stability and would be rapidly decomposed and colored under moist conditions, or in an acidic to neutral aqueous environment. It requires special techniques to avoid contact of the drug with gastric acid of the stomach. Even though stabilization of substituted benzimidazole derivatives is known in the art, there remains a need for alternate approaches to prepare stable and bio-available pharmaceutical compositions comprising esomeprazole. The present invention provides stable and bio-available pharmaceutical compositions comprising esomeprazole

Certain solid dosage forms of the present invention comprise amorphous benzimidazole compounds having PPI activity, such as the compound represented by Formula I herein described, which are unstable active ingredients, in particular, extremely unstable in acid, can be stabilized by forming a barrier coating and enteric coating layer on a drug containing core particle containing the active ingredient.

Cores may be in the form of pellets, granules or beads. The core may be acidic, alkaline, or neutral, depending on the type of formulation. The core may contain one or more pharmaceutically acceptable excipients, such as inert carriers, binders, diluents, disintegrants, lubricants/glidants, solubilizers/wetting agents, and any mixtures thereof. A core may be coated with the benzimidazole compound and one or more of binders, diluents, disintegrants, lubricants/glidants, solubilizers/wetting agents, and mixtures thereof. The core may comprise materials such as starch, microcrystalline cellulose, or sugar spheres such as nonpareil sugar seeds.

The term "acid-labile compound" means any compound, which is not stable in acidic conditions or which undergoes degradation or hydrolysis via acid or proton catalyzed reactions.

The term "excipient" means a component of a pharmaceutical product that is not an active ingredient, such as a filler, diluent, carrier, etc. The excipients that are useful in preparing a pharmaceutical composition are generally safe, non-toxic and neither biologically nor otherwise undesirable, and are acceptable for veterinary use as well as human pharmaceutical use. A "pharmaceutically acceptable excipient" as used herein includes both one and more than one such excipient.

The term 'composition' includes any 'multiple units' such as particles, powders, granules, pellets, spheroids, extrudes, or mini-tablets, comprising a drug.

In embodiments, pharmaceutical compositions comprising an acid labile drug further comprise a pharmaceutically acceptable organic base. Organic bases that may be used in the present invention include, for example, meglumine, lysine, N,N'-dibenzylethylenediamine, chloroprocain, choline, diethanolamine, ethylenediamine, procaine, and mixtures of any two or more thereof.

Organic solvents that may be used in the processes of the present invention include, but are not limited to: halogenated hydrocarbons such as dichloromethane, 1,2-dichloroethane, chloroform and carbon tetrachloride; alcohols such as methanol, ethanol, isopropyl alcohol, 1-propanol, 2-propanol, 1-butanol, 2-butanol, and t-butyl alcohol; ketones such as acetone, ethyl methyl ketone, diethyl ketone, and methyl isobutyl ketone; esters such as ethyl acetate, n-propyl acetate, n-butyl acetate and t-butyl acetate; ethers such as diethyl ether, dimethyl ether, diisopropyl ether, methyl t-butyl ether and 1,4-dioxane; nitriles such as acetonitrile and propionitrile; and any mixtures thereof.

According to embodiments of the present invention, a metal compound or an alkaline compound comprises salts of metals such as potassium, sodium, calcium, magnesium, and aluminum. Examples of metal salts include potassium hydroxide, potassium carbonate, potassium chloride, and the like, or mixtures thereof. In addition an alkaline compound may be an organic compound such as lysine or arginine or meglumine, or an antacid material such as aluminum hydroxide, calcium hydroxide or carbonate, magnesium hydroxide or carbonate, and magnesium oxide.

In embodiments, the metal compound used in the compositions of the present invention is one or more potassium salts, which can partially convert esomeprazole *in situ* into its corresponding potassium salt. In other embodiments, the metal salt used in the compositions of the present invention is one or more potassium salts, which completely convert the esomeprazole *in situ* into its corresponding potassium salt. In other embodiments, the metal salt used in the compositions of the present invention is one or more potassium salts which are present in an amount in excess of that required to convert the entire amount of esomeprazole into its corresponding potassium salt. In embodiments, the metal salt is present in an amount of about 0.1 % to about 50% by weight of the composition.

In embodiments, the active agent is contained in the compositions as esomeprazole or salt form during manufacture or storage for commercially relevant times, in an amorphous form, a crystalline form, or mixtures thereof. The esomeprazole may be present in an amorphous form or any of its crystalline forms. Esomeprazole potassium may be present in an amorphous form or any crystalline forms such as known Form A. In embodiments, the compositions of the present invention are substantially free of crystalline esomeprazole and/or esomeprazole potassium Form B, or mixtures thereof.

In embodiments, the active agent is contained in the compositions as esomeprazole or a salt form during manufacture or storage, in an amorphous form, or crystalline form, or mixtures thereof.

A binder may be any pharmaceutically acceptable, non-toxic pharmaceutically acceptable binder. In embodiments, the binder is a water soluble polymer such as a polyvinyl alcohol, a polyvinylpyrrolidone, a methylcellulose, a hydroxypropyl cellulose, a hydroxymethyl cellulose, and the like, including any mixtures thereof.

An enteric coating agent may comprise an acid resisting material which resists acidic environments up to a pH of about 5.5 or less, such as a cellulose acetate phthalate, a hydroxypropylmethyl cellulose phthalate, a polyvinyl acetate phthalate, a carboxymethylethylcellulose, Eudragit® L (poly(methacrylic acid, methylmethacrylate), 1:1 ratio (No. Av. MW 135,000--USP Type A) or Eudragit® S (poly(methacrylic acid, methylmethacrylate, 1:2 ratio (No. Av. MW 135,000--USP Type B), and any mixtures thereof. For example Eudragit® L100-55 is a 100% polymer solids product while the Eudragit® L30D-55 product is a 30% w/w aqueous dispersion of the polymer. Typical solvents that may be used to apply an enteric coating include isopropyl alcohol, acetone, methylene chloride, Isopropyl alcohol with water, and the like, and any mixtures thereof. The enteric coating is applied either directly onto drug-loaded cores or onto barrier coated cores by conventional coating techniques such as, for instance, pan coating or fluidized bed coating, using solutions of polymers in water and/or suitable organic solvents, or by using suspensions of said polymers. Generally, the enteric coating is applied in an amount of about 5% to about 100% by weight of the core or barrier coated composition.

The coatings of the present invention may comprise a plasticizer, present in amounts up to about 50% by weight of the enteric coating composition. Suitable plasticizers include acetyl triethyl citrate, dibutyl phthalate, tributyl citrate, triethyl citrate, acetyl tributyl citrate, propylene glycol, triacetin, polyethylene glycol, and diethyl phthalate. The enteric coating layer may further comprise dispersants such as talc, and colorants and pigments may also be included into the enteric coating layer.

The coating materials of the present invention may comprise lubricants such as calcium stearate, magnesium stearate, stearic acid, syloid, a coagulated aerosol of synthetic silica, pyrogenic silicon dioxide, etc.

Compositions of the present invention can be prepared using the techniques described herein, as well as other methods known to those having skill in the art.

Esomeprazole pharmaceutical compositions of the invention can be further processed into various pharmaceutical dosage forms as prepared, or can be combined with one or more pharmaceutically acceptable excipients.

In embodiments, the present invention provides pharmaceutical compositions comprising esomeprazole or salts, polymorphs, hydrates, esters, isomers, derivatives, or mixtures thereof, together with one or more pharmaceutically acceptable excipients, wherein at least one potassium salt is present as an excipient.

In embodiments, esomeprazole compositions of the present invention comprise use of esomeprazole in an amorphous form as the active agent, together with metal salt and/or at least one alkaline compound, together with one or more other excipients.

In embodiments, the present invention provides multiple unit compositions of esomeprazole, comprising: (1) a pharmacologically inert core; (2) a drug layer over the core comprising esomeprazole or its salt, at least one metal compound and/or at least one alkaline compound, and one or more pharmaceutically acceptable excipients; (3) a barrier coating layer or intermediate layer over the drug layer, comprising at least one pharmaceutically acceptable excipients; (4) an enteric layer over the barrier coating layer or intermediate layer comprising an enteric coating polymer; and (5) optionally, an overcoating layer over the enteric coating layer.

In embodiments, multiple unit compositions are provided, comprising: (1) a pharmacologically inert core; (2) a drug layer over the core comprising esomeprazole, at least one metal compound, and/or at least one alkaline compound, and one or more pharmaceutically acceptable excipient; (3) a barrier coating layer or intermediate layer over the drug layer, comprising at least one pharmaceutically acceptable excipient; (4) an enteric coating layer over the barrier coating layer or intermediate layer, comprising an enteric coating polymer; and (5) an overcoating layer over the enteric coating layer; wherein the esomeprazole composition in the form of multiple unit particles is made into a single unit dosage form such as tablet or capsule.

In embodiments, esomeprazole compositions of the present invention comprise esomeprazole in an amorphous form as the active agent, together with a metal compound and/or alkaline compound and one or more other excipients, wherein the esomeprazole is at least partially converted into an esomeprazole salt with a metal *in situ* during the manufacturing process.

In an aspect, the invention provides processes for preparing an esomeprazole composition, embodiments comprising: (1) seal coating sugar spheres using a binder suspension to obtain inert cores; (2) applying an esomeprazole drug containing layer onto the inert cores to obtain drug coated particles; (3) applying a barrier coating layer onto the drug coated particles; (4) applying an enteric coating layer onto the barrier coated particles; and (5) applying an overcoating layer onto the enteric coated particles.

In embodiments, the invention provides processes for preparing single unit dosage forms of esomeprazole, comprising: (1) seal coating sugar spheres using a binder suspension to obtain inert cores; (2) applying an esomeprazole drug containing layer onto the inert cores to obtain drug coated particles; (3) applying a barrier coating layer onto the drug coated particles; (4) applying an enteric coating layer onto the barrier coated particles; (5) optionally, applying an overcoating layer onto the enteric coated particles to obtain the overcoated multiple unit particles; (6) co-granulating the enteric coated or over coated multiple unit particles with one or more pharmaceutically acceptable excipients; and (7) making the composition thus obtained into a single unit dosage form.

In embodiments, esomeprazole compositions of the present invention are in the form of particles. In embodiments, particles according to the present invention may be in the form of powders, granules, pellets, spheroids, extrudes, mini-tablets, and the like.

The final dosage forms can be an enteric coated tablets or capsules, or in the case of enteric coated pellets or granules, these pellets or granules can be dispensed in hard gelatin capsules or sachets. The final dosage forms may further be coated with an additional layer containing pigments and/or colorants and/or moisture protecting agents.

In embodiments, esomeprazole compositions of the present invention are in the form of particles made into a unit dosage form such as tablets or capsules.

In embodiments, esomeprazole compositions of the present invention are in the form of powders or granules compressed into tablets. Compositions may be in the form of immediate release, delayed release, controlled release, or their combinations.

In embodiments, the active agent is contained in the compositions as esomeprazole or a salt during manufacture or storage, in an amorphous form, or a crystalline form, or mixtures thereof.

In embodiments, tablets of the present invention exhibit hardness of about 4 to about 25 kilopounds (KP) and friability less than about 2%.

Pharmaceutical preparations according to the invention are especially advantageous in the prevention and treatment of gastric acid-related disorders in mammals and man, including, for example, gastroesophageal reflux disease, gastritis, gastric ulcers, duodenal ulcer, etc. They are typically administered one to several times per day. A typical daily dose of the active compound will depend on various factors, such as, for example, the individual requirement of a patient, the route of administration and the disease. In general, oral and parenteral dosages will be in the range of 2.5 to 500 mg per day of active substance.

In determining bioequivalence between two products, such as a commercially-available product and a test product, pharmacokinetic studies can be conducted whereby the products are administered in a cross-over study to volunteer subjects. Blood samples are obtained at regular intervals following dosing and assayed for parent drug concentrations. For a pharmacokinetic comparison, the plasma concentration data are used to assess key pharmacokinetic parameters including area under the plasma concentration-time curve (AUC), peak plasma drug concentration (Cₘₐₓ) and time to peak plasma concentration (Tₘₐₓ).

In embodiments, the invention includes pharmaceutical formulations containing about 40 mg of esomeprazole and producing: esomeprazole Cₘₐₓ values about 50 ng/mL to about 500 ng/mL, AUC₍₀₋ₜ₎ values about 60 ng•hour/mL to about 700 ng•hour/mL, and AUC_{(0-∞)} values about 60 ng•hour/mL to about 900 ng•hour/mL; in plasma after oral administration of a single dose to healthy humans under fasting conditions.

In embodiments, the invention includes pharmaceutical formulations containing about 40 mg of esomeprazole and producing: esomeprazole Cₘₐₓ values about 30 ng/mL to about 300 ng/mL, AUC₍₀₋ₜ₎ values about 40 ng•hour/mL to about 500 ng•hour/mL, and AUC_{(0-∞)} values about 40 ng•hour/mL to about 900 ng•hour/mL; in plasma after oral administration of a single dose to healthy humans under fed conditions.

In embodiments, the invention provides processes for producing stable granules, wherein granules are processed in an environment where the relative humidity is not more than about 70%. In embodiments, the invention provides processes for producing stable granules, including drying granules at temperatures of 50 ±20°C. In embodiments, the invention provides processes for producing stable granules, wherein granules have a water content of 0.5-10% by weight, as determined using a Karl Fischer method. In embodiments, the invention provides processes for producing stable granules, wherein granules have a loss on drying in the range of about 0.02-10%, or about 0.1-5%, by weight.

In embodiments, the invention provides stable pharmaceutical compositions comprising esomeprazole or its pharmaceutically acceptable salts, or mixtures thereof, and at least one pharmaceutically acceptable excipient, wherein pH values of the compositions are less than about 14 when dispersed in water.

In embodiments, the invention provides pharmaceutical dosage forms such as multiple unit pellet systems (MUPS) tablets of esomeprazole or its pharmaceutically acceptable salts, exhibiting dispersion of tablets in 30 minutes or less, following immersion in water.

In embodiments, the invention provides pharmaceutical dosage forms, such as enteric coated tablets of esomeprazole or its pharmaceutically acceptable salts, exhibiting disintegration when tested according to the general method of Test 701 in *United States Pharmacopeia* 29, United States Pharmacopeial Convention, Rockville, Maryland, 2005 ("USP") for enteric coated tablets.

Particle size distributions in a powder can be described in terms of Dₓ values, where x is a percentage of the particles that have dimensions less than or equal to the stated size. For example, a stated size for D₅₀ indicates that half of the particles will have dimensions not exceeding that size.

In embodiments, the present invention provides esomeprazole compositions containing esomeprazole or a salt, or mixtures thereof, having particle size distributions with D₅₀ about 0.01 µm to about 100 µm, D₁₀ about 0.001 µmto about 70 µm, and D₉₀ about 0.1 µm to about 700 µm.

In embodiments, the present invention provides methods of preventing or treating diseases or disorders using compositions of the present invention comprising at least one substituted benzimidazole derivative or its salts, or mixtures thereof.

An aspect of the invention provides methods for producing a gastric acid secretion inhibitory effect to a subject in need thereof, by administering an effective amount of the pharmaceutical compositions comprising at least one substituted benzimidazole derivative or its salts or mixtures thereof.

In an aspect, the compositions of the present invention exhibit appreciable chemical storage stability.

In embodiments, the present invention provides stable compositions comprising esomeprazole or its pharmaceutically acceptable salts, which are substantially free of degradation impurities. The compositions of the present invention may contain any one or more of impurities 1, 2, 3, 4, 5, A, B, and C, or any other drug-related impurity in an amount such that any such impurity does not substantially adversely affect the safety of the composition.

In embodiments, the invention provides stable compositions comprising esomeprazole or its pharmaceutically acceptable salts, wherein levels of one or more of impurity 1, 2, 3, 4, and 5, as described herein, are less than about 5%, or less than about 1%, of the label esomeprazole content. In embodiments, the invention provides stable compositions comprising esomeprazole or its pharmaceutically acceptable salts, wherein levels of one or more of impurity A, B, and C as described herein are less than about 5%, or less than about 1%, of the label esomeprazole content. In embodiments, the invention relates to stable compositions wherein total esomeprazole-related impurities are less than about 7%, or less than about 3%, of the label esomeprazole content. Some impurities related to esomeprazole are described in the following table. The presence and concentrations of these impurities in esomeprazole and formulations containing the drug can be determined using HPLC.

| **Name** | **Identity** | **Structure** |
|---|---|---|
| Impurity 1 | N-Oxide | 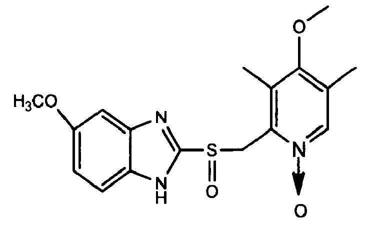 |
| Impurity 2 | Benzimidazole | 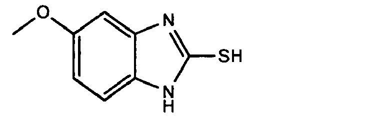 |
| Impurity 3 | Sulphone | 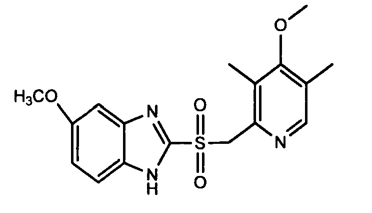 |
| Impurity 4 | Desmethoxy | 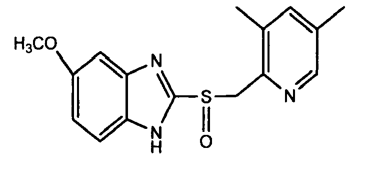 |
| Impurity 5 | Sulphide | 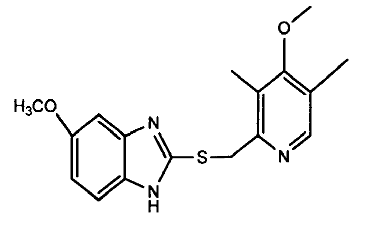 |
| Impurity A | Desmethyl dehyro | 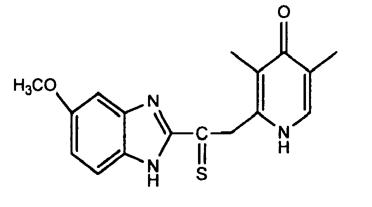 |
| Impurity B | Inter-conversion impurity | 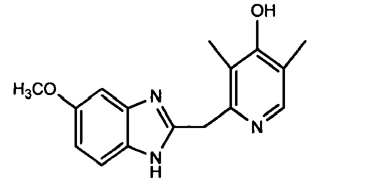 |
| Impurity C | N-Methyl omeprazole | 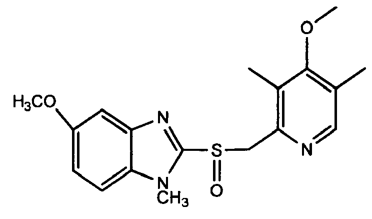 |

In embodiments, the invention provides tamper-resistant packages and/or thermo-insulated packages for pharmaceutical dosage forms of esomeprazole or its pharmaceutically acceptable salts. The compositions may be packed into blisters, strips, or containers such as plastic, glass, or metal containers. The package or packaging material optionally may contain one or more oxygen absorbants and/or dessicants.

In embodiments, the compositions of the present invention can be packaged into thermo-insulated packages, wherein aluminum blisters or HDPE bottle containers containing the composition are packaged into a suitable thermo-insulated device such as a Thermocol or an extended polystyrene package. In embodiments, the container containing the composition is double-walled wherein a hollow space between the walls is filled with air that acts as an insulator. In embodiments, either one or both of the foils used to make blisters containing the composition contains one or more layers of a desiccant.

Certain specific aspects and embodiments of the invention will be explained in more detail with reference to the following examples, being provided only for purposes of illustration, and it is to be understood that the present invention is not deemed to be limited thereto.

### EXAMPLES 1-2: Esomeprazole 20 mg and 40 mg gastro-resistant capsules.

| **Ingredient** | **mg/Capsule** | |
|---|---|---|
| | **Example 1** | **Example 2** |
| | **(40 mg)** | **(20 mg)** |
| Seal Coating | | |
| Sugar spheres | 23.81 | 11.91 |
| Hypromellose USP 2906 (Methocel® E5 Premium LV) | 0.92 | 0.46 |
| Magnesium stearate | 0.18 | 0.09 |
| Diethyl phthalate | 0.09 | 0.05 |
| Isopropyl alcohol* | q.s. | q.s. |
| Dichloromethane* | q.s. | q.s. |

| Drug Coating | | |
|---|---|---|
| Esomeprazole | 40 | 20 |
| Potassium hydroxide | 6.25 | 3.13 |
| Hydroxypropyl cellulose (Klucel® LF) | 15 | 7.5 |
| Meglumine | 6 | 3 |
| Syloid® 244 FP | 1 | 0.5 |
| Methanol* | q.s | q.s |

| Barrier Coating | | |
|---|---|---|
| Hypromellose USP 2906 (Methocel® E5 Premium LV) | 21.52 | 10.76 |
| Magnesium stearate | 4.3 | 2.15 |
| Diethyl phthalate | 2.15 | 1.08 |
| Isopropyl alcohol* | q.s. | q.s. |
| Dichloromethane* | q.s. | q.s. |

| Enteric Coating | | |
|---|---|---|
| Poly(methacrylic acid, ethyl acrylate) 1:1 (Eudragit® L100 55) | 56.83 | 38.41 |
| Talc | 28.41 | 14.21 |
| Diethyl phthalate | 5.68 | 2.84 |
| Isopropyl alcohol* | q.s. | q.s. |

| | | |
|---|---|---|
| * Evaporates during processing. | | |

### Procedure:

### SEAL COATING.

A. Preparation of coating dispersion.
   1. Hypromellose is dispersed in isopropyl alcohol (IPA) with stirring, followed by addition of dichloromethane with stirring.
   2. Magnesium stearate is homogenized in IPA for 30 minutes and then added to step 1.
   3. Diethyl phthalate is dissolved in dichloromethane, added to step 1, and stirred for 15 minutes.
   4. The dispersion is filtered through a #80 mesh sieve.
B. Seal coating.
   5. Sugar spheres are loaded into a fluid bed coater (FBC) and warmed for 10 minutes to a product temperature of 40°C.
   6. The dispersion prepared in step 4 is sprayed onto sugar spheres to achieve a weight gain of 5%.
   7. Seal coated pellets are dried in the FBC for 20 minutes at a product temperature of 40°C.

### DRUG COATING.

A. Preparation of drug-binder dispersion.
   8. Potassium hydroxide is dissolved in methanol with stirring.
   9. Esomeprazole is dissolved in methanol with stirring.
   10. Klucel® LF, meglumine and Syloid® 244 FP are dispersed in methanol with stirring.
   11. The solution of step 8 is added slowly to step 9 with stirring until complete precipitation occurs. The stirring is continued for 1 hour.
   12. The dispersion of step 10 is added to the dispersion of step 11 and stirring is continued for 30 minutes.
   13. The dispersion is passed through a #80 mesh sieve.
B. Drug coating.
   14. Seal coated pellets of step 7 are loaded into a FBC.
   15. The drug-binder dispersion of step 13 is sprayed at a temperature of 30-35°C.
   16. After spraying, the pellets are dried with a product temperature of 32°C for 15-30 minutes.

### BARRIER COATING.

A. Preparation of barrier coating dispersion.
   17. Hypromellose is dispersed in IPA with stirring, followed by addition of dichloromethane with stirring.
   18. Magnesium stearate is homogenized in IPA for 30 minutes and then added to step 17.
   19. Diethyl phthalate is dissolved in dichloromethane, added to step 17, and stirred for 15 minutes.
   20. The dispersion is filtered through a #80 mesh sieve.
B. Barrier coating.
   21. Drug coated pellets of step 16 are loaded into a FBC.
   22. The barrier coating dispersion of step 20 at a product temperature of 30-35°C is then sprayed onto the pellets.
   23. After spraying, pellets are dried at a product temperature of 32°C for 15-30 minutes.

### ENTERIC COATING.

A. Preparation of enteric coating dispersion.
   24. Eudragit® L100 55 is dissolved in IPA with stirring.
   25. Talc is homogenized in IPA for 30 minutes and then added to step 24.
   26. Diethyl phthalate is dissolved in IPA, added to step 24 and stirred for 15 minutes.
   27. The dispersion is filtered through a #80 mesh sieve.
B. Enteric coating.
   28. Barrier coated pellets of step 23 are loaded into a FBC.
   29. The enteric coating dispersion of step 27 at product temperature of 30-35°C is sprayed onto pellets.
   30. Enteric coated pellets are filled into capsules.

The resulting capsules of Example 1 and Nexium® 40 mg capsules are tested for their dissolution properties using USP apparatus 2 (paddle), with 100 rpm stirring in 0.1 N HCl for 120 minutes, then in pH 6.8 phosphate buffer for the remainder of the test. Results are shown in Table 1.

**Table 1**

| **Minutes** | **Cumulative % of Drug Released** | |
|---|---|---|
| | **Nexium® 40 mg** | **Example 1** |
| 0 | 0 | 0 |
| 10 | 64 | 92 |
| 15 | 90 | 102 |
| 20 | 95 | 102 |
| 30 | 96 | 101 |
| 45 | 93 | 100 |

### EXAMPLES 3-5: Esomeprazole 40 mg gastro-resistant capsules.

| **Ingredient** | **mg/Capsule** | | |
|---|---|---|---|
| | **Example 3** | **Example 4** | **Example 5** |
| Seal Coating | | | |
| Sugar spheres | 71.43 | 57.14 | 23.81 |
| Hypromellose USP 2906 (Methocel® E5 Premium LV) | 2.75 | 2.2 | 0.92 |
| Magnesium stearate | 0.55 | 0.44 | 0.18 |
| Diethyl phthalate | 0.27 | 0.22 | 0.09 |
| Isopropyl alcohol* | q.s. | q.s. | q.s. |
| Dichloromethane* | q.s. | q.s. | q.s. |
| Total | 75 | 60 | 25 |

| Drug Coating | | | |
|---|---|---|---|
| Esomeprazole | 40 | 40 | 40 |
| Potassium hydroxide | 6 | 6 | 6 |
| Hydroxypropyl cellulose (Klucel® LF) | 10.2 | 15 | 15 |
| Meglumine | 5 | 5 | 5 |
| Syloid® 244 FP | -- | -- | 1 |
| Methanol* | q.s. | q.s. | q.s. |
| Total | 136.2 | 126 | 92 |

| Barrier Coating | | | |
|---|---|---|---|
| Hypromellose USP 2906 (Methocel® E5 Premium LV) | 31.43 | 29.08 | 21.23 |
| Magnesium stearate | 6.29 | 5.82 | 4.25 |
| Diethyl phthalate | 3.14 | 2.91 | 2.12 |
| Isopropyl alcohol* | q.s. | q.s. | q.s. |
| Dichloromethane* | q.s. | q.s. | q.s. |
| Total | 177.06 | 163.81 | 119.6 |

| Enteric Coating | | | |
|---|---|---|---|
| Poly(methacrylic acid, ethyl acrylate) 1:1 (Eudragit® L100 55) | 55.33 | 102.38 | 74.75 |
| Talc | 27.67 | 51.19 | 37.38 |
| Diethyl phthalate | 5.53 | 10.24 | 7.48 |
| Isopropyl alcohol* | q.s. | q.s. | q.s. |
| Total | 265.59 | 327.62 | 239.21 |

| | | | |
|---|---|---|---|
| * Evaporates during processing. | | | |

Gastro-resistant capsules are prepared in a similar manner to that described for Examples 1 and 2.

Capsules prepared in Examples 3 and 5 are subjected to storage at 30°C and 65% RH in closed HDPE bottles containing a 2 g silica gel desiccant pouch, for various durations. It is found that the capsules containing esomeprazole or its salts remain substantially stable. Impurity analysis results are shown in Table 2, where values are percentages of the label omeprazole content.

**Table 2**

| **Impurity** | **Example 3** | | | | **Example 5** |
|---|---|---|---|---|---|
| | **Initial** | **1 Month** | **2 Months** | **3 Months** | **1 Month** |
| N-oxide (impurity 1) | 0 | 0 | 0 | 0 | 0 |
| Benzimidazole (impurity 2) | 0.013 | 0.032 | 0.048 | 0.05 | 0.073 |
| Sulphone (impurity 3) | 0 | 0.02 | 0.019 | 0.02 | 0.042 |
| Desmethoxy (impurity 4) | 0 | 0 | 0 | 0.029 | 0 |
| Sulphide (Impurity 5) | 0.017 | 0.014 | 0.018 | 0.018 | 0.016 |
| Desmethyl dehydro esomeprazole (impurity A) | 0 | 0.031 | 0.041 | 0.046 | 0.102 |
| Interconversion (Impurity B) | 0 | 0.025 | 0.077 | 0.062 | 0.065 |
| N-Methyl omeprazole (Impurity C) | 0 | 0.035 | 0.069 | 0.12 | 0.078 |
| Highest unidentified | 0.036 | 0 | 0.106 | 0.145 | 0.095 |
| Total | 0.079 | 0.157 | 0.608 | 0.588 | 0.661 |

### EXAMPLE 6: Esomeprazole potassium 40 mg capsules.

| **Ingredient** | **mg/Capsule** |
|---|---|
| Seal Coating | |
| Sugar spheres | 23.81 |
| Hypromellose USP 2906 (Methocel® E5 Premium LV) | 0.92 |
| Magnesium stearate | 0.18 |
| Diethyl phthalate | 0.09 |
| Isopropyl alcohol* | q.s. |
| Dichloromethane* | q.s. |
| Total | 25 |

| Drug Coating | |
|---|---|
| Esomeprazole potassium | 44.4 |
| Potassium hydroxide | 6 |
| Hydroxypropyl cellulose (Klucel® LF) | 15 |
| Meglumine | 5 |
| Syloid® 244 FP | 1 |
| Methanol* | q.s. |
| Total | 96.4 |

| Barrier Coating | |
|---|---|
| Hypromellose USP 2906 (Methocel® E5 Premium LV) | 22.25 |
| Magnesium stearate | 4.45 |
| Diethyl phthalate | 2.22 |
| Isopropyl alcohol* | q.s. |
| Dichloromethane* | q.s. |
| Total | 125.32 |

| Enteric Coating | |
|---|---|
| Poly(methacrylic acid, ethyl acrylate) 1:1 (Eudragit® L100 55) | 54.83 |
| Talc | 27.41 |
| Diethyl phthalate | 5.48 |
| Isopropyl alcohol* | q.s. |
| Total | 213.04 |

| | |
|---|---|
| * Evaporates during processing. | |

Capsules are prepared in a similar manner to the procedure described for Examples 1 and 2.

### EXAMPLE 7: Esomeprazole 40 mg tablets.

| **Ingredient** | **mg/Tablet** |
|---|---|
| Seal Coating | |
| Sugar spheres | 23.81 |
| Hypromellose USP 2906 (Methocel® E5 Premium LV) | 0.92 |
| Magnesium stearate | 0.18 |
| Diethyl phthalate | 0.09 |
| Isopropyl alcohol* | q.s. |
| Dichloromethane* | q.s. |
| Total | 25 |

| Drug Coating | |
|---|---|
| Esomeprazole | 40 |
| Potassium hydroxide | 6.5 |
| Hydroxypropyl cellulose (Klucel® LF) | 15 |
| Meglumine | 5 |
| Syloid® 244 FP | 1 |
| Methanol* | q.s. |
| Total | 92.5 |

| Barrier Coating | |
|---|---|
| Hypromellose USP 2906 (Methocel® E5 Premium LV) | 21.35 |
| Magnesium stearate | 4.27 |
| Diethyl phthalate | 2.13 |
| Isopropyl alcohol* | q.s. |
| Dichloromethane* | q.s. |
| Total | 120.25 |

| Enteric Coating | |
|---|---|
| Poly(methacrylic acid, ethyl acrylate) 1:1 (Eudragit® L100 55) | 56.37 |
| Talc | 28.18 |
| Diethyl phthalate | 5.64 |
| Isopropyl alcohol* | q.s. |
| Total | 210.44 |

| Overcoating | |
|---|---|
| PEG 6000 | 31.57 |
| Methanol* | q.s. |
| Methylene chloride* | q.s. |
| Total | 242.01 |

| Blending, Lubrication, and Compression | |
|---|---|
| Mannitol (Pearlitol® SD 200) | 375.54 |
| Microcrystalline cellulose (Avicel® PH 200) | 160.95 |
| Crospovidone (Polyplasdone® XL) | 45 |
| Silicon dioxide (Syloid® 244 FP) | 9 |
| PEG 600 | 22.5 |
| Copovidone (Kollidon® VA 64) | 36 |
| Magnesium stearate | 9 |
| Total | 900 |

| Moisture Barrier Coating | |
|---|---|
| Opadry® AMB OY-B29000 | 27 |
| Water* | q.s. |
| Total | 927 |

| | |
|---|---|
| * Evaporates during processing. | |

### Procedure:

Enteric coated pellets are prepared according to the general procedure described for Examples 1 and 2. The enteric coated pellets are overcoated using PEG 6000. The overcoated pellets are mixed with mannitol (Pearlitol® SD 200), microcrystalline cellulose (Avicel® PH 200), crospovidone (Polyplasdone® XL), silicon dioxide (Syloid® 244 FP), PEG 600, and copovidone (Kollidon VA 64) for 15 minutes. The mixture is then blended with magnesium stearate and compressed into tablets.

### EXAMPLE 8: Esomeprazole 40 mg tablets.

| **Ingredient** | **mg/Tablet** |
|---|---|
| Dry Mixing | |
| Microcrystalline cellulose (Avicel PH 200) | 77.5 |
| Mannitol (Pearlitol® SD 200) | 90 |
| Total | 167.5 |

| Granulation | |
|---|---|
| Esomeprazole | 40 |
| Potassium hydroxide | 6 |
| Hydroxypropyl cellulose (Klucel® LF) | 10 |
| Meglumine | 5 |
| Methanol* | q.s. |
| Total | 228.5 |

| Blending | |
|---|---|
| Crospovidone | 12.5 |
| Syloid® 244 FP | 1 |
| Lubrication and Compression | |
| Magnesium stearate | 2.5 |
| Total | 242 |

| Barrier Coating | |
|---|---|
| Hypromellose USP 2906 (Methocel® E5 Premium LV) | 27.92 |
| Magnesium stearate | 5.58 |
| Diethyl phthalate | 2.79 |
| Isopropyl alcohol* | q.s. |
| Dichloromethane* | q.s. |
| Total | 278.3 |

| Enteric Coating | |
|---|---|
| Poly(methacrylic acid, ethyl acrylate) 1:1 (Eudragit® L100 55) | 34.79 |
| Talc | 17.39 |
| Diethyl phthalate | 3.48 |
| Isopropyl alcohol* | q.s. |
| Total | 333.96 |

| Moisture Barrier Coating | |
|---|---|
| Opadry® AMB OY-B29000 | 10.02 |
| Water* | q.s. |
| Total | 343.98 |

| | |
|---|---|
| * Evaporates during processing. | |

### Procedure:

Mix microcrystalline cellulose and mannitol in a FBC and granulate with a dispersion containing esomeprazole, potassium hydroxide, hydroxypropyl cellulose (Klucel® LF), meglumine, and methanol. Dry the granulated mass and pass through #40 mesh sieve. Mix the granules with crospovidone and Syloid® 244 FP in a blender for 15 minutes, then add magnesium stearate and blend for 5 minutes. Compress the blend into tablets. The tablets are sequentially coated with a barrier coating, an enteric coating, and a moisture barrier coating.

### EXAMPLE 9: Esomeprazole 40 mg capsules.

| **Ingredient** | **mg/Capsule** |
|---|---|
| Drug Coating | |
| Core | |
| Sugar spheres 25/30 mesh | 75 |

| Dry Mix | |
|---|---|
| Mannitol (Pearlitol®) | 18.75 |
| L-Hydroxypropyl cellulose PC-LH31 | 18.75 |

| Drug-binder Solution | |
|---|---|
| Esomeprazole | 40 |
| Klucel® LF | 3 |
| Potassium hydroxide | 6.5 |
| Meglumine | 5 |
| Methanol* | q.s. |
| Total | 167 |

| Barrier Coating | |
|---|---|
| Hydroxypropyl methylcellulose E5 LV Premium | 38.54 |
| Magnesium stearate | 7.71 |
| Diethyl phthalate | 3.85 |
| Isopropyl alcohol* | q.s. |
| Methylene chloride* | q.s. |
| Total | 217.1 |

| Enteric Coating | |
|---|---|
| Eudragit® L100-55 | 67.81 |
| Talc | 33.91 |
| Diethyl phthalate | 6.78 |
| Isopropyl alcohol* | q.s. |
| Total | 325.6 |

| | |
|---|---|
| * Evaporates during processing. | |

Procedure: Sugar spheres are loaded into a centrifugal coating granulator and sprayed with the drug-binder dispersion, while a mixture of mannitol and LHPC is added simultaneously. The drug loaded pellets are dried in a rapid dryer at 35°C for 30 minutes. The pellets are coated sequentially with a barrier coating and an enteric coating, and filled into capsules.

### EXAMPLES 10-11: Esomeprazole 40 mg capsules.

| **Ingredient** | **mg/Capsule** | |
|---|---|---|
| | **Example 10** | **Example 11** |
| Seal Coating | | |
| Sugar spheres | 57.14 | 23.81 |
| Hypromellose USP 2906 (Methocel® E5 Premium LV) | 2.2 | 0.92 |
| Magnesium stearate | 0.44 | 0.18 |
| Diethyl phthalate | 0.22 | 0.09 |
| Isopropyl alcohol* | q.s. | q.s. |
| Dichloromethane* | q.s. | q.s. |
| Total | 60 | 25 |

| Drug Coating | | |
|---|---|---|
| Esomeprazole | 40 | 40 |
| Potassium hydroxide | 6 | -- |
| Hydroxypropyl cellulose (Klucel® LF) | 15 | 15 |
| Meglumine | 5 | 5 |
| Syloid® 244 FP | -- | 1 |
| Methanol* | q.s. | q.s. |
| Total | 126 | 86 |

| Barrier Coating | | |
|---|---|---|
| Hypromellose USP 2906 (Methocel® E5 Premium LV) | 29.08 | 19.85 |
| Magnesium stearate | 5.82 | 3.97 |
| Diethyl phthalate | 2.91 | 1.98 |
| Isopropyl alcohol* | q.s. | q.s. |
| Dichloromethane* | q.s. | q.s. |
| Total | 163.81 | 111.80 |

| Enteric Coating | | |
|---|---|---|
| Poly(methacrylic acid, ethyl acrylate) 1:1 (Eudragit® L100 55) | 102.38 | 48.91 |
| Talc | 51.19 | 24.46 |
| Diethyl phthalate | 10.24 | 4.89 |
| Isopropyl alcohol* | q.s. | q.s. |
| Total | 327.62 | 190.06 |

| | | |
|---|---|---|
| *Evaporates during processing. | | |

Procedure: capsules are prepared in a similar manner to that described for Examples 1 and 2.

Table 3 illustrates the results of a study to determine impurity concentrations after storage, for compositions prepared with and without an metal compound, potassium hydroxide (KOH), using the compositions of Examples 10 and 11. Drug coated pellets are stored unpackaged at 40°C and 75% RH for three days. It is found that the drug coated pellets containing the esomeprazole remain substantially more stable when a metal compound, such as potassium hydroxide, is added to the formulation.

**Table 3**

| **Impurity** | **Drug Coated Pellets** | |
|---|---|---|
| | **With KOH** | **Without KOH** |
| N-oxide (Impurity 1) | ND | ND |
| Benzimidazole (Impurity 2) | 0.117 | 0.167 |
| Sulphone (Impurity 3) | 0.029 | 0.045 |
| Desmethoxy (Impurity 4) | 0.056 | 0.041 |
| Sulphide (Impurity 5) | 0.084 | 0.172 |
| Desmethyl dehydro esomeprazole (Impurity A) | 0.106 | 0.501 |
| Interconversion (Impurity B) | 0.011 | 0.033 |
| N-Methyl omeprazole (Impurity C) | 0.054 | 0.208 |
| Highest unidentified | 0.049 | 0.154 |
| Total | 0.552 | 1.997 |

| | | |
|---|---|---|
| ND = not detected. | | |

### EXAMPLES 12-13: Esomeprazole 20 mg and 40 mg gastro-resistant tablets.

| **Ingredient** | **mg/Tablet** | |
|---|---|---|
| | **Example 12** | **Example 13** |
| Seal Coating | | |
| Sugar spheres | 23.81 | 11.91 |
| Hypromellose 2910 (Methocel® E 5 LV) | 0.92 | 0.46 |
| Magnesium stearate | 0.18 | 0.09 |
| Diethyl phthalate | 0.09 | 0.05 |
| Isopropyl alcohol* | q.s. | q.s. |
| Dichforomethane* | q.s. | q.s. |
| Total | 25 | 12.5 |

| Drug Coating | | |
|---|---|---|
| Esomeprazole | 40 | 20 |
| Potassium hydroxide | 6.25 | 3.13 |
| Hydroxypropyl cellulose (Klucel® LF) | 15 | 7.5 |
| Meglumine | 6 | 3 |
| Syloid 244 FP | 1 | 0.5 |
| Methanol* | q.s. | q.s. |
| Total | 93.25 | 46.63 |

| Barrier Coating | | |
|---|---|---|
| Hypromellose 2910 (Methocel® E 5 LV) | 21.52 | 10.76 |
| Magnesium stearate | 4.3 | 2.15 |
| Diethyl phthalate | 2.15 | 1.08 |
| Isopropyl alcohol* | q.s. | q.s. |
| Dichloromethane* | q.s. | q.s. |
| Total | 121.22 | 60.61 |

| Enteric Coating | | |
|---|---|---|
| Poly(methacrylic acid, ethyl acrylate) 1:1 (Eudragit® L100 55) | 56.82 | 28.41 |
| Talc | 28.41 | 14.21 |
| Diethyl phthalate | 5.68 | 2.84 |
| Isopropyl alcohol* | qs | qs |
| Total | 212.14 | 106.07 |

| Overcoating | | |
|---|---|---|
| PEG 6000 | 31.82 | 15.91 |
| Dichloromethane* | q.s. | q.s. |
| Methanol* | q.s. | q.s. |
| Total | 243.96 | 121.98 |

| Lubrication | | |
|---|---|---|
| Talc | 1.22 | 0.61 |
| Total | 245.18 | 122.59 |

| Co-granulation | | |
|---|---|---|
| Lactose monohydrate Lactose monohydrate 187.825 (impalpable) | 187.825 | 93.91 |
| Microcrystalline cellulose (Avicel® PH101) | 255 | 127.5 |
| Croscarmellose sodium (Ac-Di-Sol®) | 34 | 17 |
| PVP K-30 | 38 | 19 |
| Water* | q.s. | q.s. |

| Blending | | |
|---|---|---|
| PEG 6000 | 50 | 25 |
| Crospovidone Type-A (Kollidon® CL) | 34 | 17 |

| Lubrication | | |
|---|---|---|
| Sodium stearyl fumarate | 6 | 3 |
| | Compression | |
| Total | 850 | 425 |

| Moisture Barrier Coating | | |
|---|---|---|
| Opadry® AMB Pink | 25.5 | 12.75 |
| Water* | qs | qs |
| Total | 875.5 | 437.75 |

| | | |
|---|---|---|
| * Evaporates during processing. | | |

Procedure: Enteric coated pellets are prepared in a similar manner to that described for Examples 1 and 2. Further, the enteric coated pellets are overcoated, mixed with a disintegrant and cushioning agent, lubricated and compressed into tablets. The tablets are further provided with a moisture barrier coating.

## Claims

1. A pharmaceutical formulation comprising: (a) a pharmacologically inert core; (b) a drug layer over the core comprising a benzimidazole drug, at least one metal compound and/or at least one alkaline compound, and one or more pharmaceutically acceptable excipients; (c) a barrier coating layer or intermediate layer over the drug layer, comprising at least one pharmaceutically acceptable excipient; (d) an enteric layer over the barrier coating layer or intermediate layer comprising an enteric coating polymer; and (e) optionally, an overcoating layer over the enteric coating layer.

2. The pharmaceutical formulation according to claim 1, wherein the metal compound comprises potassium hydroxide, potassium carbonate, potassium chloride, or any combinations thereof, and the alkaline compound is meglumine.

3. The pharmaceutical formulation according to claim 1 or 2, wherein the benzimidazole drug is at least partially converted into a salt with the metal compound during manufacture.

4. The pharmaceutical formulation according to any of claims 1-3, wherein the benzimidazole drug comprises esomeprazole.

5. The pharmaceutical formulation according to any of claims 1-3, wherein the benzimidazole drug is esomeprazole, esomeprazole potassium, or a mixture of esomeprazole and esomeprazole potassium.

6. The pharmaceutical formulation according to claim 5, wherein the esomeprazole, esomeprazole potassium, or mixture of esomeprazole and esomeprazole potassium is in an amorphous form, a crystalline form, or any mixtures thereof.

7. The pharmaceutical formulation according to any of claims 1-6, being in the form of particles, powders, granules, pellets, spheroids, extrudates, or mini-tablets.

8. The pharmaceutical formulation according to any of claims 1-6, being in the form of multiple unit particles made into a unit dosage form such as a tablet or capsule.

9. The pharmaceutical formulation according to claim 8, wherein the tablet or capsule comprises overcoated multiple unit particles together with one or more pharmaceutically acceptable excipients.

10. A process for preparing a pharmaceutical formulation, comprising: (a) seal coating sugar spheres with a binder suspension to obtain inert cores; (b) applying a benzimidazole drug-containing layer onto the inert cores to obtain drug coated particles; (c) applying a barrier coating layer onto the drug coated particles; (d) applying an enteric coating layer onto the barrier coated particles; and (e) optionally applying an overcoating layer onto the enteric coated particles.

11. A process according to claim 10, further comprising: (f) co-granulating enteric coated or overcoated multiple unit particles with one or more pharmaceutically acceptable excipients; and (g) making the composition thus obtained into a unit dosage form such as a tablet or capsule.

12. A process according to claim 10 or 11, wherein a benzimidazole drug is esomeprazole.
